# EUROPÄISCHE PATENTSCHRIFT

(11) **EP 0 698 383 B1**
(45) Veröffentlichungstag und Bekanntmachung des Hinweises auf die Patenterteilung: **12.04.2000**
(21) Anmeldenummer: 95113350.3
(22) Anmeldetag: 25.08.1995
(51) Int. Cl.: A61F 13/15

(54) **Wiederverwendbare Saugmattenunterlage**
Reusable absorbant mat support
Support de matte absorbante reutilisable

(30) Priorität: 27.08.1994 DE 9413900 U
(43) Veröffentlichungstag der Anmeldung: 28.02.1996
(73) Patentinhaber: GERMED Gesellschaft für medizinischen Bedarf mbH, 21493 Schwarzenbek (DE)
(72) Erfinder: Ernst, Gerhard, D-21527 Kollow (DE)
(74) Vertreter: Serwe, Karl-Heinz, Dipl.-Ing.

(56) Entgegenhaltungen:
- DE-U- 9 212 761

## Beschreibung

Die Erfindung betrifft eine wiederverwendbare Saugmattenunterlage zur Aufnahme von Feuchtigkeit, bestehend aus einer unteren Schicht aus einem Textilgewebe aus Polyester, dessen Oberseite für Feuchtigkeit undurchlässig mit Polyurethan beschichtet ist, aus einer mittleren, Feuchtigkeit aufnehmenden Saugschicht aus einem Polyester-Vlies und aus einer oberen Feuchtigkeit durchlassenden Deckschicht aus einem textilen Polyester-Mikrofasergewebe.

Aus dem deutschen Gebrauchsmuster 92 12 761.4 des Anmelders ist eine derartige Saugmattenunterlage bekannt, die sich in der Praxis wegen ihres geringen Gewichtes und der damit verringerten Reinigungskosten bewährt hat.

Die Erfahrung hat jedoch gezeigt, daß nach häufigem Waschen sich die Saugschicht aus Polyester-Vlies stellenweise verschiebt, so daß die Saugmattenunterlage unterschiedliche Dicke aufweist. Dies ist nach häufigem Waschen auch dann der Fall, wenn die obere Deckschicht aus dem textilen Polyester-Mikrofasergewebe und die mittlere Saugschicht aus Polyester-Vlies durch über Kreuz oder in Wellen laufende Nähte miteinander verbunden sind.

Die Aufgabe der Erfindung besteht darin, eine Saugmattenunterlage der eingangs genannten Art derart weiterzubilden, daß auch nach häufigen Waschen kein Verschieben und Verrubbeln der mittleren Saugschicht aus Polyester-Vlies auftritt.

Diese Aufgabe wird dadurch gelöst, daß zwischen der mittleren Saugschicht und der unteren Schicht eine weitere Schicht aus einem textilen Polyester-Mikrofasergewebe angeordnet ist.

Vorteilhaft sind die obere Deckschicht, die mittlere Saugschicht und die weitere Schicht aus einem textilen Polyester-Mikrofasergewebe durch über Kreuz oder in Wellen verlaufende Nähte miteinander verbunden.

Vorteilhaft sind die drei oberen Schichten mit der unteren Schicht an den Rändern zusammengekettelt.

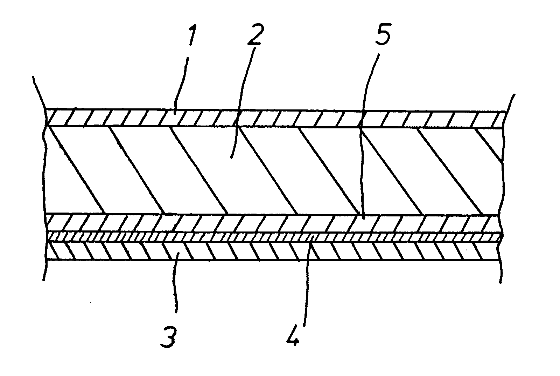

Die Erfindung ist in der Zeichnung beispielhaft dargestellt. Die Zeichnung zeigt die neuerungsgemäße Saugmattenunterlage in einem teilweisen Schnitt.

Bei einer erfindungsgemäßen Saugmattenunterlage besteht eine obere Deckschicht 1 aus einem textilen Polyesther-Mikrofasergewebe, eine mittlere Saugschicht 2 aus einem Polyesther-Vlies und eine untere Schicht 3 aus einem Textilgewebe aus Polyester, die auf ihrer Oberseite 4 feuchtigkeitsundurchlässig mit Polyurethan beschichtet ist.

Wie die Zeichnung weiter zeigt, ist zwischen der mittleren Saugschicht 2 und unteren Schicht 3, 4 eine weitere Schicht 5 aus einem textilen Polyester-Mikrofasergewebe angeordnet.

Die obere Deckschicht 1 ist für Feuchtigkeit durchlässig, so daß die durchtretende Feuchtigkeit in der mittleren Saugschicht 2 aufgenommen werden kann. Die weitere Schicht 5 aus einem textilen Polyester-Mikrofasergewebe ist gleichfalls feuchtigkeitsdurchlässig, jedoch verhindert die untere Schicht 3, 4 ein Durchtreten von Feuchtigkeit.

Durch die weitere Schicht 5 aus einem textilen Polyester-Mikrofasergewebe wird gewährleistet, daß die Saugschicht 2 aus Polyester-Vlies auch nach häufigem Waschen nicht verrutscht, so daß die Saugmattenunterlage stets die gleiche Dicke aufweist. Dies gilt insbesondere dann, wenn die oberen Schichten 1, 2 und 5 durch über Kreuz oder in Wellen verlaufende Nähte miteinander verbunden sind.

Die vorgeschlagene erfindungsgemäße Saugmattenunterlage ist kochfest bis 95° C und kann der normalen Krankenhauswäsche zugeteilt werden. Da keine Baumwolle Verwendung findet, ist ein geringerer Einsatz von Waschmitteln und weniger Spülwasser erforderlich.

## Patentansprüche

1. Wiederverwendbare Saugmattenunterlage zur Aufnahme von Feuchtigkeit, bestehend aus einer unteren Schicht aus einem Textilgewebe aus Polyester, dessen Oberseite für Feuchtigkeit undurchlässig mit Polyurethan beschichtet ist, aus einer mittleren, Feuchtigkeit aufnehmenden Saugschicht aus einem Polyester-Vlies und aus einer oberen Feuchtigkeit durchlässigen Deckschicht aus einem textilen Polyester-Mikrofasergewebe, dadurch gekennzeichnet, daß zwischen der mittleren Saugschicht (2) aus Polyester-Vlies und der unteren Schicht (3, 4) eine weitere Schicht (5) aus einem textilen Polyester-Mikrofasergewebe angeordnet ist.

2. Saugmattenunterlage nach Anspruch 1, dadurch gekennzeichnet, daß die obere Deckschicht (1), die mittlere Saugschicht (2) und die weitere Schicht (5) aus Polyester-Mikrofasergewebe durch über Kreuz oder in Wellen verlaufende Nähte miteinander verbunden sind.

3. Saugmattenuterlage nach Anspruch 2, dadurch gekennzeichnet, daß die beiden oberen Schichten (1, 2 und 5) mit der unteren Schicht (3, 4) an den Rändern zusammengekettelt sind.

## Claims

1. Reusable absorbent mat support for absorbing moisture, consisting of a lower layer of a woven fabric made from polyester, the upper face of which is coated with polyurethane so as to render it impermeable to moisture, a central moisture-absorbing absorbent layer made from non-woven polyester, and an upper moisture-permeable top layer made from a woven polyester microfibre fabric, characterised in that arranged between the central absorbent layer (2) made from non-woven polyester and the lower layer (3, 4) there is a further layer (5) made from a woven polyester microfibre fabric.

2. Absorbent mat support according to claim 1, characterised in that the upper top layer (1), the central absorbent layer (2), and the further layer (5) of polyester microfibre fabric are joined to one another by seams which run crosswise or in wavy lines.

3. Absorbent mat support according to claim 2, characterised in that the two upper layers (1, 2 and 5) are looped together at the edges with the lower layer (3, 4).

## Revendications

1. Support de natte absorbante pour emmagasiner de l'humidité, composé d'une couche inférieure réalisée en une toile de polyester, dont la surface supérieure est rendue imperméable en étant revêtue par du polyuréthane, d'une couche intermédiaire absorbante d'humidité réalisée par une texture de polyester, et d'une couche supérieure perméable à l'humidité réalisée en un tissu de microfibres de polyester, caractérisé en ce qu'une couche additionnelle (5) en tissu de microfibres de polyester est insérée entre la couche intermédiaire (2) absorbante en texture de polyester, et la couche inférieure (3, 4).

2. Support de natte absorbante selon la revendication 1, caractérisé en ce que la couche supérieure (1), la couche intermédiaire (2) absorbante, et la couche additionnelle (5) en tissu de microfibres de polyester sont reliées entre elles par des coutures continues en croix ou ondulées.

3. Support de natte absorbante selon la revendication 2, caractérisé en ce que les deux couches supérieures (1, 2, et 5) sont reliées aux bords de la couche inférieure (3, 4) par des points à chaînette.
